# EUROPEAN PATENT APPLICATION

(11) **EP 2 682 751 A1**
(43) Date of publication of application: **08.01.2014**
(21) Application number: 12174897.4
(22) Date of filing: 04.07.2012
(51) Int. Cl.: G01N 33/68

(54) **Method for diagnosing psychiatric disorders in a human subject assessing the concentration of SP1, SP3 and SP4 proteins in a biological sample**

(71) Applicant: Parc Sanitari Sant Joan de Déu, 08830 Sant Boi de Llobregat (ES); Cibersam, 28007 Madrid (ES)
(72) Inventor: Ramos Josemaria, Belén, 08840 Viladecans (ES); Fusté Boadella, Monserrat, 08037 Barcelona (ES); Pinacho García, Raquel, 08038 Barcelona (ES); Haro Abad, Josep Maria, 08037 Barcelona (ES); Villalta Gil, Victoria, 08172 Sant Cugat del Vallés (ES); Roca Cassús, Mercedes, 08950 Esplungues de Llobregat (ES)
(74) Representative: Carvajal y Urquijo, Isabel

(57) **Abstract**

A method and a kit for diagnosing psychiatric disorders in a human subject, comprising assessing the concentration of SP1, SP3 and SP4 proteins in a biological sample obtained from said human subject, determining whether at least one of the markers is above or below standard values from healthy subjects, wherein said markers are selected from the group composed by SP1 protein concentration, SP4 protein concentration, sum of SP1, SP3 and SP4 protein concentrations and sum of SP1, SP4 protein concentrations and diagnosing psychiatric disorders on said human subject evaluating the result of previous step. The invention also relates to a method and a kit for diagnosing severe positive and negative symptoms of schizophrenia in a human subject.

## Description

### FIELD OF THE INVENTION

The present invention is related to human necessities, medical science. Specifically, this invention focuses on diagnostics of psychiatric disorders in a human subject, evaluating the SP1, SP3 and SP4 protein concentrations in a biological sample and diagnosing psychiatric disorders by comparison of said protein concentrations with protein concentration values from healthy subjects. Said psychiatric disorders are first episode psychosis, schizophreniform disorder, schizoaffective disorder, schizophrenia, delusional disorder or psychotic disorder not otherwise specified, or bipolar disorder I.

### BACKGROUND OF THE INVENTION

Nowadays, the diagnosis of severe psychiatric disorders is based on clinical symptoms and there are no biological tests to this aim. An initial diagnosis of psychiatric disorders is performed at early stages only based on clinical parameters which will be refined after a follow-up of the patient evolution for months or years. In the case of subjects with a First Episode Psychosis (FEP), the initial diagnosis will be changed to a diagnosis with more affective components (i.e. Bipolar Disorder) or with more psychotic features (i.e. schizophrenia). A biological/molecular test that helps to facilitate a more rapid and appropriate diagnosis at early stages will lead to a better treatment.

The understanding of pathologic mechanisms of psychiatric disorders is currently limited. Schizophrenia (SZ) constitutes a complex disorder with a high diversity in symptoms. Symptoms of patients with SZ have been classified in different symptom dimensions: negative (i.e. lack of volition, poor or absent social functioning, blunted affect), positive (hallucinations, delusions), affective (anxiety, depression) and cognitive (disorganization of thought processes). Although positive symptoms usually lead to diagnosis, they are often preceded by negative and cognitive symptomatology of varying severity. Each subject has a specific symptom profile which suggests the existence of multiple schizophrenias and the consideration of SZ as a syndrome instead of a unique entity. Hypodopaminergic activity in prefrontal cortex and other cortical areas has been associated to more severe negative symptoms and cognitive impairment in schizophrenia, whereas hyperdopaminergic activity in subcortical areas has been associated to positive symptoms. However, the detailed underlying molecular mechanisms of different type of symptoms remain unclear. The advance in this field has been slow and fragmentary in part due to the barrier of studying SZ as a homogeneous disease. This lack of detailed brain molecular information linked to clinical symptoms has not allowed progressing enough in the development of specific drug therapies that target a particular symptom.

It is known that there are structural and functional abnormalities in the prefrontal cortex, hippocampus and the cerebellum of SZ subjects. The cerebellum contributes to psychological alterations and, its impairment in schizophrenia correlates with cerebellar neurologic defects and negative symptoms suggesting that cerebellum may contribute to negative symptoms in addition to a hypodopaminergic activity in the prefrontal cortex. Alterations in the hippocampus have been associated to the onset of schizophrenia, poor clinical outcome, duration of the illness as well as the implication of this area in neuropsychological defects and symptoms of schizophrenia.

Although several molecular alterations have been identified in the prefrontal cortex and hippocampus of patients, limited information is available about the changes that occur in the cerebellum and no information has been reported linked to symptomatology for the three brain areas. The transcription factor SP1 (Specificity Protein 1) and SP4 are members of the Sp family which control a large variety of genes involved in diverse biological processes. Sp4 and Sp1 transcription factors share high homology in the DNA binding domain. Sp4 can function as either an activator or a repressor of transcription, thus, Sp4 and Sp1 may function independently, cooperate or even compete to regulate genes through a common DNA element. An example of this functional diversity in neuronal cells comes from our study that identified that Sp4 has an essential role for dendritic development while *Sp1* did not show any effect in dendritic morphogenesis. Thus, SP transcription factors have overlapping, but in many cases, non-overlapping functions and changes in SP4 do not imply the same changes in SP1 and the same biological effects. Reduced Sp4 expression in mice induces behavioural defects associated with psychiatric disorders and genetic variations of SP4 human gene have been associated with SZ. However, no data has been provided in human brain or peripheral tissues for SP4 in schizophrenia or early psychosis. In addition, altered gene expression of SP1 transcription factor in SZ has been observed in brain and lymphocytes of chronic patients with schizophrenia (Ben-Shachar, D. et al., Sp1 expression is disrupted in schizophrenia; a possible mechanism for the abnormal expression of mitochondrial complex I genes, NDUFV1 and NDUFV2. PLoS One, 2007. 2(9): p. e817). Said document discloses that Sp1 mRNA increased in lymphocytes of chronic schizophrenic patients as compared with controls.

However, the present invention establishes that SP1 protein levels are unexpected in comparison to the prior art, as SP1 decreased in peripheral mononuclear blood cells in early psychosis compared to a reference sample of healthy individuals. In addition, the present invention establishes that SP4 and SP1 protein levels are unexpected in comparison to the prior art, as SP4 increased in hippocampus in schizophrenia patients as compared to a reference sample of healthy individuals.

### DESCRIPTION OF THE INVENTION

The present invention is a method for diagnosing psychiatric disorders in a human subject, herewith, method of the invention, comprising:
a) assessing the concentration of SP1, SP3 and SP4 proteins in a biological sample obtained from said human subject,
b) determining whether at least one of the markers is above or below standard values from healthy subjects, wherein said markers are selected from the group composed by SP1 protein concentration, SP4 protein concentration, sum of SP1, SP3 and SP4 protein concentrations and sum of SP1, SP4 protein concentrations,
c) diagnosing psychiatric disorders on said human subject evaluating the result of step b).

Another aspect is the method of the invention, where said biological sample is obtained from the group composed by peripheral mononuclear blood cells, prefrontal cortex biopsy, cerebellum biopsy and hippocampus biopsy.

Another aspect is the method of the invention, where said peripheral mononuclear blood cells are lymphocytes.

Another aspect is the method of the invention, where said psychiatric disorders are first episode psychosis, schizophreniform disorder, schizoaffective disorder, schizophrenia, delusional disorder or psychotic disorder not otherwise specified, or bipolar disorder I.

Another aspect is the method of the invention, where a decrease of said markers levels compared to standard values from healthy subjects in peripheral mononuclear blood cells indicates the presence of a psychiatric disorder in said human subject.

Another aspect is the method of the invention, where a decrease of said markers levels compared to standard values from healthy subjects in hippocampus indicates the presence of schizophrenia in said human subject.

Another aspect is a kit for performing the method of the invention, comprising appropriate reagents for the quantification of the concentration of SP1, SP3 and SP4 proteins in a biological sample obtained from said human subject.

Another aspect is a method for diagnosing severe positive and negative symptoms of schizophrenia in a human subject, comprising:
a) assessing the concentration of SP1 and SP4 proteins in a prefrontal cortex or cerebellum sample obtained from said human subject,
b) determining whether SP1 or SP4 are above or below standard values from subjects with schizophrenia,
c) diagnosing severe positive or negative symptoms of schizophrenia on said human subject evaluating the result of step b).

In the present invention, the term "positive symptoms of schizophrenia" refers to hallucinations and delusions and the term "negative symptoms of schizophrenia" refers to lack of volition, poor or absent social functioning and blunted affect. Said positive and negative symptoms of schizophrenia are used to classify patients with schizophrenia and to design an appropriate therapy from this classification.

Another aspect of the invention is the method for diagnosing severe positive and negative symptoms of schizophrenia in a human subject, where an increase of SP1 and SP4 protein levels compared to standard values from subjects with schizophrenia in prefrontal cortex indicates the presence of severe positive symptoms of schizophrenia.

Another aspect of the invention is the method for diagnosing severe positive and negative symptoms of schizophrenia in a human subject, where a decrease of SP1 and SP4 protein levels compared to standard values from subjects with schizophrenia in cerebellum indicates the presence of severe negative symptoms of schizophrenia.

Another aspect of the invention is the method for diagnosing severe positive and negative symptoms of schizophrenia in a human subject, where a decrease of SP1 protein level compared to standard values from subjects with schizophrenia in hippocampus indicates the presence of severe negative symptoms of schizophrenia.

Another aspect of the invention is a kit for performing the method for diagnosing severe positive and negative symptoms of schizophrenia in a human subject, comprising appropriate reagents for the quantification of the concentration of SP1 and SP4 proteins in a biological sample obtained from said human subject.

### BRIEF DESCRIPTION OF THE FIGURES

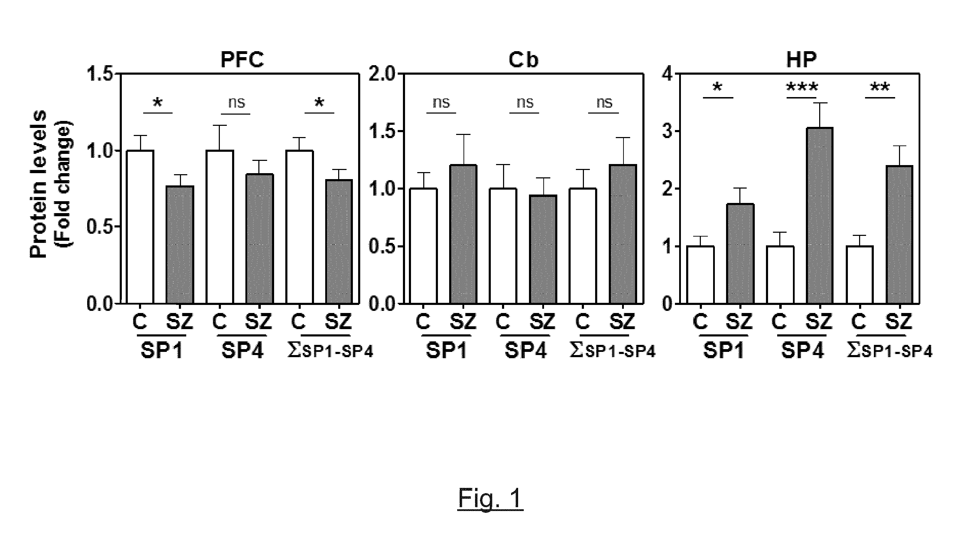

Figure 1 shows densitometric analysis of protein levels in postmortem extracts of prefrontal cortex (PFC), cerebellum (Cb) and hippocampus (HP) from control (C, n=15 for PFC and Cb; n=12 for HP), and schizophrenia (SZ, n=20 for PFC and Cb, n=15 for HP) subjects determined by immunoblotting for SP1, SP4, and GAPDH. SP levels were normalized to GAPDH values, and a reference healthy control sample. Levels were determined in at least two independent analyses. SP1 and SP4 relative values were also summed and normalized to control group (∑SP1-SP4). Mean ± standard error of the mean is shown for each group. Statistical analysis was performed using unpaired t test except of SP1 levels in hippocampus assed by Mann-Whitney test. (p<0.05-*, p<0.01-**, p<0.001-***, n.s., not significant).

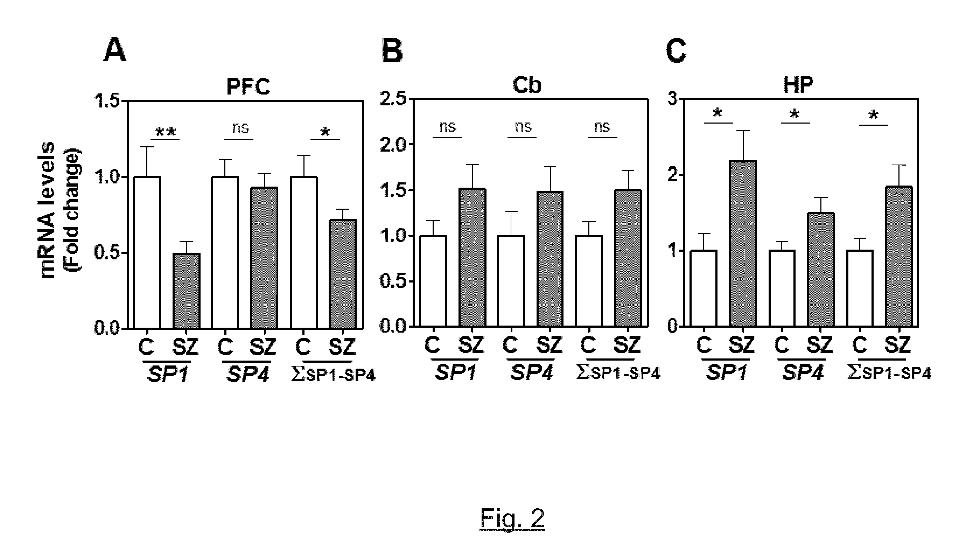

Figure 2 shows RT-qPCR analysis of SP1 and SP4 mRNA levels in postmortem extracts of prefrontal cortex (PFC), cerebellum (Cb) and hippocampus (HP) from control (C, n=15 for PFC and Cb; n=11 for HP), and schizophrenia (SZ, n=20 for PFC and Cb, n=15 for HP) subjects. SP levels were referenced to a TBP and GUSB normalization factor in cerebellum and prefrontal cortex and to GUSB in hippocampus, and a healthy control reference sample. Levels were determined in at least two independent analyses performed in duplicates. SP1 and SP4 relative values were also summed and normalized to control group (∑SP1-SP4). Mean ± standard error of the mean is shown for each group. Statistical analysis was performed using unpaired t test. (p<0.05-*, p<0.01-**, n.s., not significant).

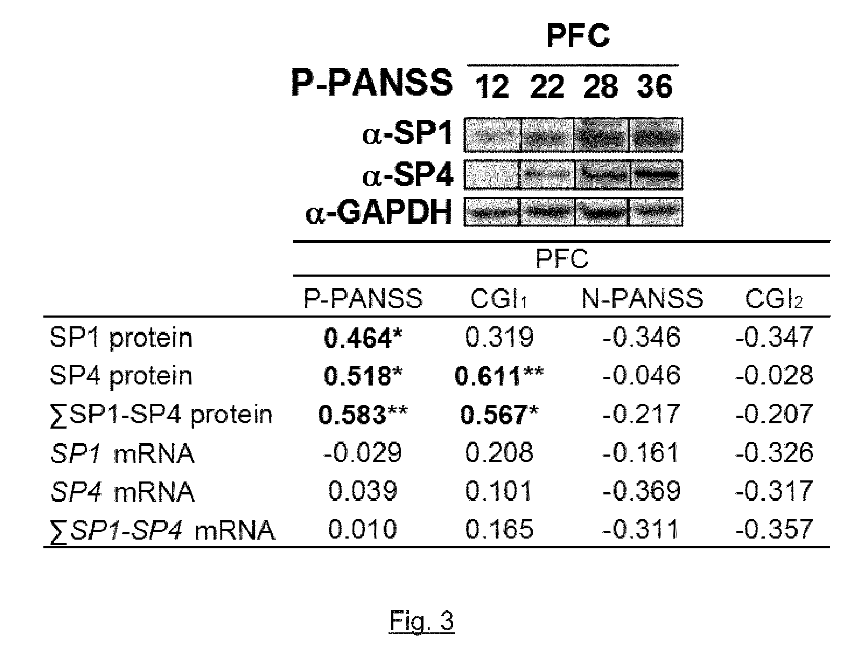

Figure 3 shows representative images for SP and GAPDH immunoblots of schizophrenia subjects (n=20) postmortem extracts of prefrontal cortex (PFC) and their scores for positive (P-PANSS) symptoms measured by the Positive and Negative Syndrome Scale (PANSS). The table below shows Pearson correlations of SP factors protein and mRNA levels or their sum (ΣSP1-SP4) with positive (P-PANSS and CGI₁) and negative symptoms (N-PANSS and CGI₂) measured by the PANSS and the Clinical Global Impression-Schizophrenia Scale (CGI-SCH). (p<0.05-*, p<0.01-**).

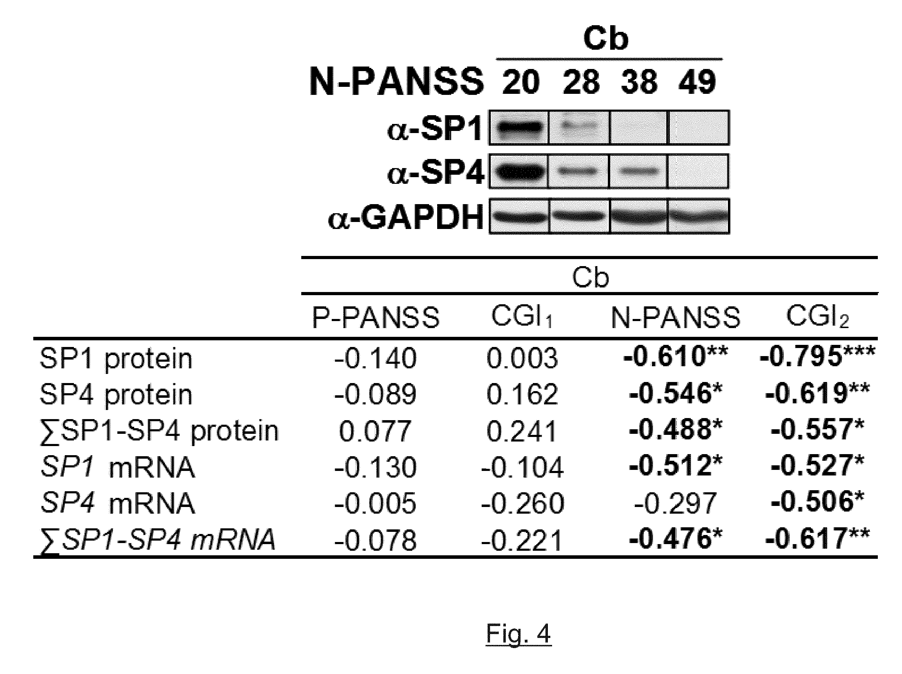

Figure 4 shows representative images for SP and GAPDH immunoblots of schizophrenia subjects (n=20) postmortem extracts of cerebellum (Cb) and their scores for negative (N-PANSS) symptoms measured by the Positive and Negative Syndrome Scale (PANSS). The table below shows Pearson correlations of SP factors protein and mRNA levels or their sum (ΣSP1-SP4) with positive (P-PANSS and CGI₁) and negative (N-PANSS and CGI₂) symptoms measured by the PANSS and the Clinical Global Impression-Schizophrenia Scale (CGI-SCH). (p<0.05-*, p<0.01-**, p<0.001-***).

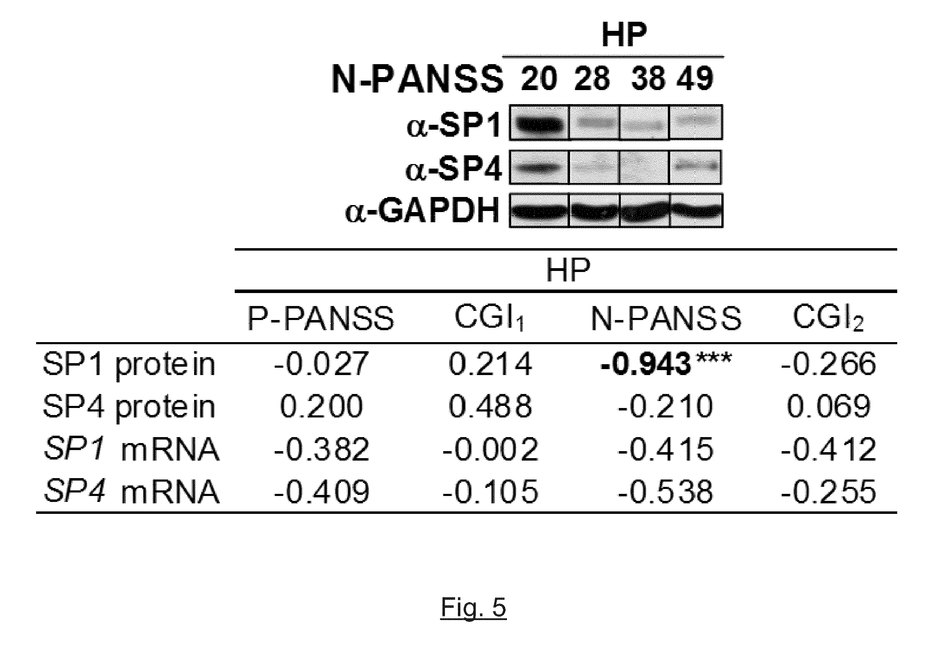

Figure 5 shows representative images for SP and GAPDH immunoblots of schizophrenia subjects (n=11, PANSS; n=16, CGI-SCH) postmortem extracts of hippocampus (HP) and their scores for negative (N-PANSS) symptoms measured by the Positive and Negative Syndrome Scale (PANSS). The table below shows Spearman correlations of SP factors protein and mRNA levels with positive (P-PANSS and CGI₁) and negative symptoms (N-PANSS and CGI₂) measured by the PANSS and the Clinical Global Impression-Schizophrenia Scale (CGI-SCH). (p<0.001-***).

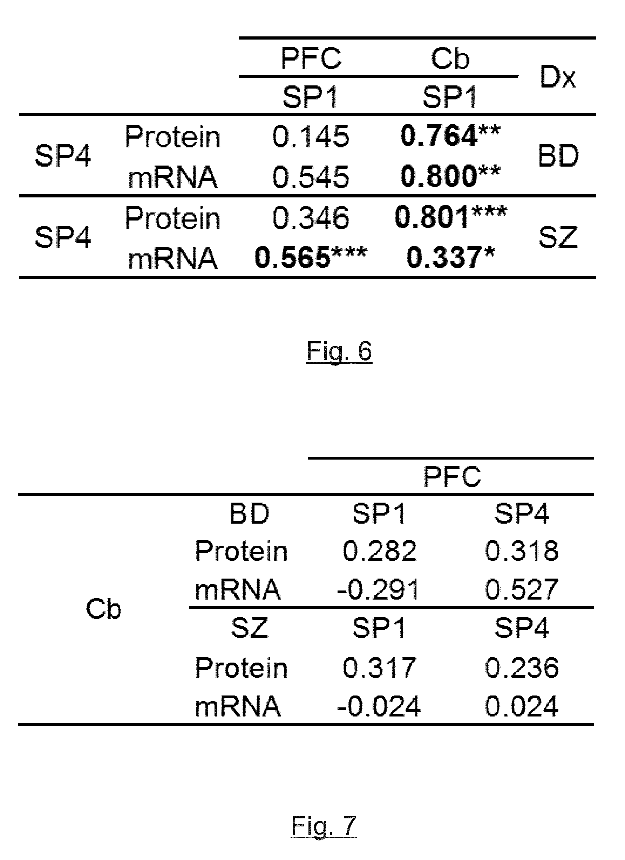

Figure 6 shows Spearman correlations of SP factors protein and mRNA expression levels in postmortem extracts of prefrontal cortex (PFC) and cerebellum (Cb) from bipolar disorders (BD, n=11) and schizophrenia (SZ, n=20) subjects. (p<0.05-*, p<0.01- **, p<0.001-***).
Figure 7 shows Spearman correlations comparing SP factors protein and mRNA levels in postmortem extracts of prefrontal cortex (PFC) and cerebellum (Cb) in bipolar (BD, n=11) and schizophrenia (SZ, n=20) subjects. None of the associations were significantly different.

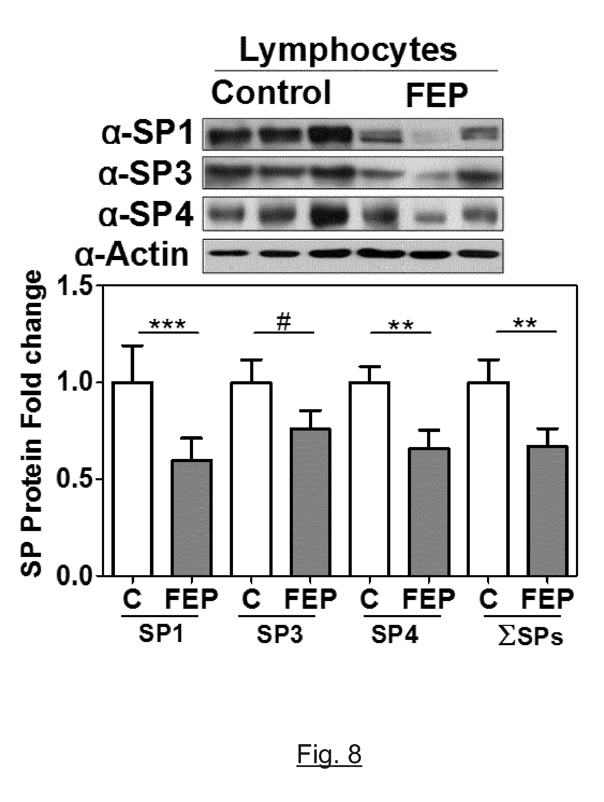

Figure 8 shows representative images for SP1, SP3 and SP4 and actin inmunoblots in peripheral mononuclear blood cells in First Episode Psychosis subjects (FEP) and control individuals. The graph bellow shows the relative abundance of SP1, SP3 and SP4 protein levels and the sum of them (ΣSPs) in control (n=13) and FEP (n=13) groups. Values of each individual SP protein were normalized to actin. Mean ± standard error of the mean is shown for each group. Paired t-test analysis was performed (p<0.001- ***, p<0.01- **, p=0,0681- #).

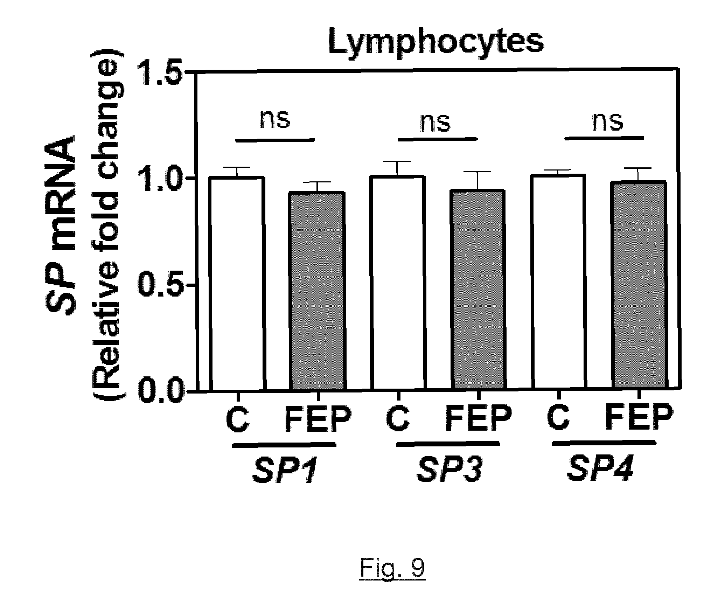

Figure 9 shows the relative abundance of SP1, SP3 and SP4 mRNA levels in control (n=13) and FEP (n=13) groups. Values of each individual SP protein were normalized to TBP. Mean ± standard error of the mean is shown for each group. Paired t-test analysis was performed (ns- not significant).

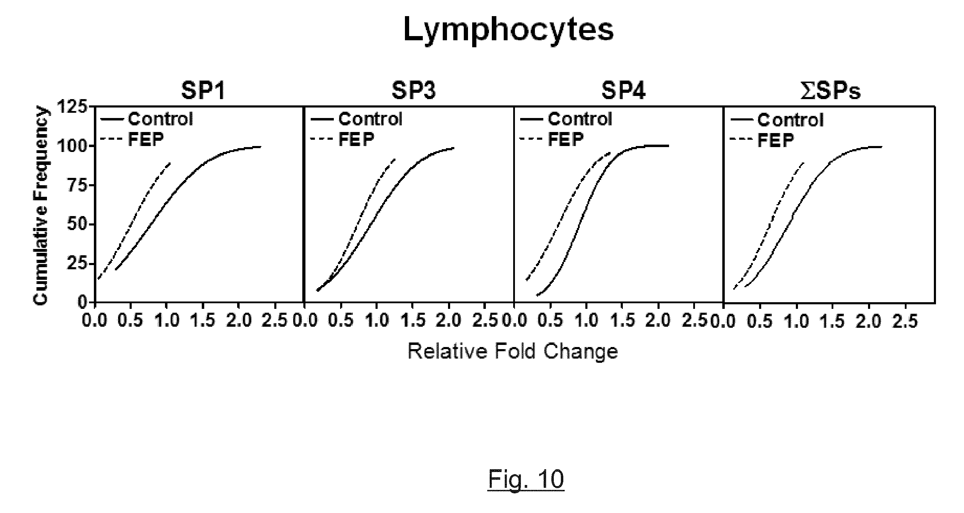

Figure 10 shows the Gaussian cumulative frequency distribution for lymphocyte SP1 SP3 and SP4 protein levels and the sum of them in First Episode Psychosis (n=14) and control (n=15) groups.

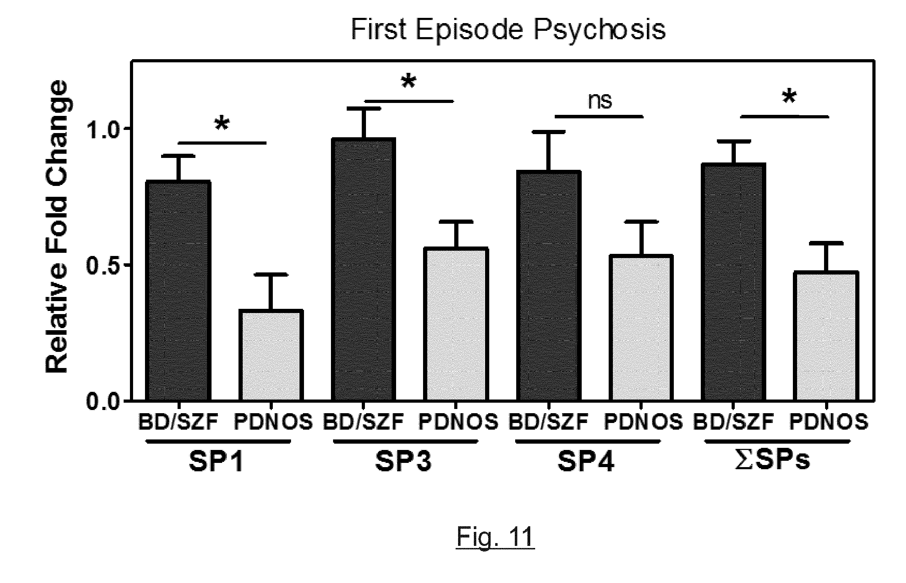

Figure 11 shows quantification of lymphocyte SP1, SP3 and SP4 protein levels and the sum of them in First Episode Psychosis participants with an initial diagnosis of Psychotic Disorder Not Otherwise Specified (PDNOS) (n=7) compared to First Episode Psychosis subjects with a specific diagnosis (n=7) (Schizophreniform disorder or Bipolar Disorder (SZF/BD)). Graph shows SP1, SP3 and SP4 protein levels and the sum of them (ΣSPs) in both diagnosis groups. Values of each individual SP protein were normalized to actin. Mean ± standard error of the mean is shown for each group. Mann Whitney test was performed (p <0.05- *, ns- not significant).

### EXAMPLES OF THE INVENTION

### Example 1. Schizophrenia cohort for postmortem brain study

A cohort of subjects with schizophrenia (SZ) were selected, n=20 for prefrontal cortex (PFC) and cerebellum (Cb) and n=15 for hippocampus (Hp), compared to control individuals, n=15 for PFC and Cb and n=12 for Hp. SZ and control groups were matched by age, gender, *postmortem* interval and brain pH. Protein and mRNA were extracted from PFC, Cb and Hp. Premortem Clinical evaluation was performed in SZ brain donors by Positive and Negative Syndrome Scale (PANSS) and Clinical Global Impression Scales for Schizophrenia (CGI-SCH).

### Example 2. SP1 and SP4 characterization in postmortem brain in schizophrenia

To explore the possible alteration of SP4 and SP1 transcription factors protein expression in brain in schizophrenia, we analyzed the protein levels of SP4 and also SP1 in several *postmortem* brain areas of chronic subjects with schizophrenia (SZ). We observed a decrease of SP1 protein levels in prefrontal cortex of SZ group, while no changes were found for SP4 protein (Figure 1A). Both SP1 and SP4 were increased in hippocampus, but not in cerebellum, in SZ (Figure 1B and 1C). The sum of SP1 and SP4 protein levels was decreased in prefrontal cortex and increased in hippocampus in schizophrenia group (Figure 1A and 1C). We further explored the changes of SP1 and SP4 mRNA levels in these brain areas. We found a decrease in SP1, but not SP4, mRNA in prefrontal cortex in SZ (Figure 2A). In addition, we observed an increase of SP1 and SP4 mRNA in hippocampus in SZ group and no changes in cerebellum for both factors (Figure 2B and 2C). The sum of SP1 and SP4 gene expression levels was decreased in prefrontal cortex and increased in hippocampus in schizophrenia group (Figure 2A and 2C). We next examined the levels of SP1 and SP4 related to clinical symptoms (positive and negative) in our SZ cohort. Both SP1 and SP4 protein levels and the sum of them in prefrontal cortex associated to positive symptoms and not to negative symptoms (Figure 3). In cerebellum, SP1, SP4 proteins and the sum of their levels associated to negative symptoms evaluated by PANSS and ICG-SCH scales (Figure 4). In addition, cerebellar SP1 and SP4 mRNA also significantly correlated to negative symptoms (Figure 4). Moreover, only SP1 protein levels also associated to negative symptoms evaluated by PANSS scale in hippocampus (Figure 5). Therefore, these results show a different brain-regional regulation of SP proteins in schizophrenia compared to controls and linked to a different type of symptomatology in schizophrenia.

### Example 3. Differences and similarities between SP1 and SP4 expression in subjects with a psychiatric disorder

To further study the differences and similarities of SP1 and SP4 expression in psychiatric disorders, we analysed the correlations for both factors in a particular brain area in schizophrenia and bipolar disorder. We identified that SP1 protein did not associate to SP4 protein levels in the prefrontal cortex in both disorders suggesting that each factor is controlled by independent mechanisms in psychiatric disorders (Figure 6). However, in the cerebellum both factors are highly correlated which reflect a common regulation in this brain area. Since SP proteins may have overlapping and non-overlapping functions, with this analysis we better characterized their similarities and their differences for psychiatric disorders. Moreover, the change of SP1 or SP4 in cerebellum did not reflect a change of these factors in prefrontal cortex (Figure 7) suggesting that Sp factors are brain specific-regionally regulated in psychiatric disorders.

### Example 4. Selection of First Episode Psychosis subjects for peripheral mononuclear blood cells analysis

Subjects that suffered their first psychotic episode were selected (n=14). Control healthy subjects matched by age and gender were also recruited (n=15). A blood sample was obtained soon after their clinical stabilization and, peripheral mononuclear blood cells (PMBCs) isolation was performed after less than an hour from the time of blood extraction. Then, mRNA and protein were extracted from PMBC in control and First Episode Psychosis subjects (FEP).

### Example 5. SP1 and SP4 proteins are reduced in lymphocytes in First Episode Psychosis subjects

Protein levels of the transcription factor SP1 and SP4 in PMBCs compared to healthy individuals were analyzed. We found a significant decrease of SP1 and SP4 protein levels in First Episode Psychosis subjects and only a tendendy to be significantly reduced for SP3 protein (Figure 8). In addition, the sum of SP1, SP3 and SP4 proteins was also significantly reduced in First Episode Psychosis (Figure 8). However, mRNA SP factor individual levels were not reduced in First Episode Psychosis subjects (Figure 9). We also compared the cumulative frequency curves for SP1, SP3, SP4 protein levels and the sum of them in our control and First Episode Psychosis populations. Lower levels for the three SP proteins and the sum of them were detected in First Episode Psychosis population compared to a healthy population (Figure 10). We further investigated the levels of SP1, SP3 and SP4 protein levels and the sum of them in the different diagnosis groups. Diagnosis was evaluated once the FEP participants reached clinical stability, as assessed by their assigned psychiatrist before discharge. We identified a significant decrease of SP1 proteins levels in subjects with a Psychotic Disorder Not Otherwise Specified diagnosis group compared to the specific diagnosis group (Schizophreniform disorder or Bipolar Disorder) (Figure 11). Thus, these results show that SP1, SP3, SP4 and the sum of them are peripheral biomarkers of earlier stages of the psychotic disorders.

## Claims

1. Method for diagnosing psychiatric disorders in a human subject, comprising:
a) assessing the concentration of SP1, SP3 and SP4 proteins in a biological sample obtained from said human subject,
b) determining whether at least one of the markers is above or below standard values from healthy subjects, wherein said markers are selected from the group composed by SP1 protein concentration, SP4 protein concentration, sum of SP1, SP3 and SP4 protein concentrations and sum of SP1, SP4 protein concentrations and
c) diagnosing psychiatric disorders on said human subject evaluating the result of step b).

2. Method according to claim 1, **characterized in that** said biological sample is obtained from the group composed by peripheral mononuclear blood cells, prefrontal cortex biopsy, cerebellum biopsy and hippocampus biopsy.

3. Method according to claim 2, **characterized in that** said peripheral mononuclear blood cells are lymphocytes.

4. Method according to any of claims 1 to 3, **characterized in that** said psychiatric disorders are first episode psychosis, schizophreniform disorder, schizoaffective disorder, schizophrenia, delusional disorder or psychotic disorder not otherwise specified, or bipolar disorder I.

5. Method according to any of claims 1 to 4, **characterized in that** a decrease of said markers levels compared to standard values from healthy subjects in peripheral mononuclear blood cells indicates the presence of a psychiatric disorder in said human subject.

6. Method according to any of claims 1 to 4, **characterized in that** a decrease of said markers levels compared to standard values from healthy subjects with schizophrenia in hippocampus indicates the presence of schizophrenia in said human subject.

7. Kit for performing the method according to claims 1 to 6, comprising appropriate reagents for the quantification of the concentration of SP1, SP3 and SP4 proteins in a biological sample obtained from said human subject.

8. Method for diagnosing severe positive and negative symptoms of schizophrenia in a human subject, comprising:
a) assessing the concentration of SP1 and SP4 proteins in a prefrontal cortex or cerebellum sample obtained from said human subject,
b) determining whether SP1 or SP4 are above or below standard values from subjects with schizophrenia and
c) diagnosing severe positive or negative symptoms of schizophrenia on said human subject evaluating the result of step b).

9. Method according to claim 8, **characterized in that** an increase of SP1 and SP4 protein levels compared to standard values from subjects with schizophrenia in prefrontal cortex indicates the presence of severe positive symptoms of schizophrenia.

10. Method according to claim 8, **characterized in that** a decrease of SP1 and SP4 protein levels compared to standard values from subjects with schizophrenia in cerebellum indicates the presence of severe negative symptoms of schizophrenia.

11. Method according to claim 8, **characterized in that** a decrease of SP1 protein level compared to standard values from subjects with schizophrenia in hippocampus indicates the presence of severe negative symptoms of schizophrenia.

12. Kit for performing the method according to claims 8 to 11, comprising appropriate reagents for the quantification of the concentration of SP1 and SP4 proteins in a biological sample obtained from said human subject.
